# EUROPÄISCHE PATENTSCHRIFT

(11) **EP 0 399 485 B1**
(45) Veröffentlichungstag und Bekanntmachung des Hinweises auf die Patenterteilung: **01.12.1993**
(21) Anmeldenummer: 90109746.9
(22) Anmeldetag: 22.05.1990
(51) Int. Cl.: C07D 319/12

(54) **Verfahren zur Herstellung von D,L-Lactid**
Process for the production of D,L-lactide
Procédé pour la production de D,L-lactide

(30) Priorität: 26.05.1989 DE 3917178
(43) Veröffentlichungstag der Anmeldung: 28.11.1990
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Hess, Joachim, Dr., Dipl.-Chem., D-6530 Bingen (DE); Müller, Klaus Robert, Dr., Dipl.-Ing. u. Chem., D-6507 Ingelheim am Rhein (DE); Müller, Manfred, Dr., Dipl.-Chem., D-6101 Biskenbach (DE)

(56) Entgegenhaltungen:
- DD-A- 261 362
- DE-A- 3 632 103
- DE-A- 3 708 915
- DE-A- 3 820 299

## Beschreibung

Die Erfindung betrifft ein Verfahren zur Herstellung von D,L-Lactid, welches ohne weitere Reinigungsschritte in Polymerisationsreaktionen eingesetzt werden kann.

Polymere des D,L-Lactids finden in einer Vielzahl von Einsatzgebieten Verwendung - so z.B. bei der Herstellung von bioabbaubaren chirurgischen Hilfsmitteln - wie chirurgisches Nahtmaterial, orthopädische Stifte, Gefäßimplantate etc. Bei der Herstellung der Polymerisate kann - in Abhängigkeit von den gewünschten Eigenschaften - das D,L-Lactid allein zum Poly-D,L-lactid oder zusammen mit anderen Monomeren zu den entsprechenden Copolymerisaten umgesetzt werden. Die Eigenschaften der resultierenden Polymerisate werden dabei erheblich von der Reinheit des eingesetzten D,L-Lactids beeinflußt.

Bisher sind zur Herstellung von D,L-Lactid lediglich Verfahren bekannt, in denen als Ausgangsmaterial racemische Milchsäure eingesetzt wird.

Diese Verfahren weisen jedoch u.a. den schwerwiegenden Nachteil auf, daß bei der Umsetzung von racemischer Milchsäure zu D,L-Lactid zwangsläufig ein Anteil von mindestens 50 % an unerwünschtem meso-Lactid anfällt. Die Abtrennung des meso-Lactids vom D,L-Lactid gelingt nur unter Anwendung aufwendiger, teurer und verlustreicher Verfahren (mehrfaches Umkristallisieren aus verschiedenen Lösungsmitteln, Extraktion, Sublimation).

Ein von E. Jungfleisch und M. Godchot entwickeltes Verfahren [C.R. Hebd. Séances Acad. Sci. 142 (1906) 639] geht von D- und L-Lactid aus, welche nach Co-Kristallisation das gewünschte D,L-Lactid liefern. Dieses Verfahren leidet jedoch unter dem Nachteil, daß als Lösungsmittel der leicht brennbare Diethylether in einem Gewichtsverhältnis von 70:1 eingesetzt werden muß (bei technischen Verfahren liegt das Verhältnis von Lösungsmittel zum Feststoff im Regelfall in einem Bereich von 2:1 bis 10:1). Ein weiterer Nachteil dieses Verfahrens besteht darin, daß das so hergestellte D,L-Lactid nicht ohne zusätzliche aufwendige Reinigungsschritte polymerisiert werden kann.

Es ist somit die Aufgabe der vorliegenden Erfindung, ein Verfahren zur Verfügung zu stellen, welches die Herstellung von D,L-Lactid unter industriellen Produktionsbedingungen ermöglicht, das ohne zusätzliche Reinigungsoperationen zu Poly-D,L-lactid bzw. den entsprechenden Copolymeren polymerisiert werden kann.

Erfindungsgemäß wird diese Aufgabe dadurch gelöst, daß die Co-Kristallisation von D-Lactid und L-Lactid in Lösungsmitteln bzw. Lösungsmittelgemischen durchgeführt wird, die das azeotrope Abdestillieren von Wasser, Oligomeren und von restlicher Milchsäure sowie von Lösungsmittelresten, die die spätere Polymerisationsreaktion nachteilig beeinflussen, ermöglichen und, daß man D- und L-Lactid anschließend zusammen auskristallisieren läßt und das Co-Kristallisat isoliert.

Im Gegensatz zu den bisher bekannten Verfahren zur Herstellung von D,L-Lactid erhält man bei der Anwendung des erfindungsgemäßen Verfahrens ein D,L-Lactid, welches unter industriellen Bedingungen hergestellt ohne weitere Reinigungsoperationen in Polymerisationsreaktionen eingesetzt werden kann.

Als Ausgangsmaterialien werden D(+)-Lactid und L(-)-Lactid eingesetzt.

Als Lösungsmittel und Lösungsmittelgemische finden Lösungsmittel Verwendung, die mit Wasser ein azeotropes Gemisch bilden können und welche die Co-Kristallisation nicht nachteilig beeinflussen. - Beispiele derartiger Azeotrope sind dem Fachmann bekannt [Timmermans, The Physico-Chemical Constants of Binary Systems in Concentrated Solutions, Interscience, New York N.Y., 1959-1960 und Ergänzungswerk 1965 ff; Azeotropic Data, Advances in Chemistry, Series No. 6 und No. 35, American Chemical Society, Washington D.C., 1952 und 1962].

Bevorzugt wird Toluol als Lösungsmittel eingesetzt.

Das bzw. die eingesetzte(n) Lösungsmittel werden zweckmäßigerweise vor der Co-Kristallisation - z.B. azeotrop - entwässert.

L(-)-Lactid und D(+)-Lactid werden zweckmäßigerweise unter Feuchtigkeitsausschluß jeweils in einem Teil des Lösungsmittels bzw. der Lösungsmittelmischung gelöst. Vorzugsweise werden L(-)- und D(+)-Lactid in jeweils der Hälfte der Gesamtmenge des eingesetzten Lösungsmittels (bzw. der eingesetzten Lösungsmittelmischung) gelöst.

Die Lösungen werden zweckmäßigerweise heiß filtriert und anschließend miteinander vermischt. Darauf wird ein Teil des Lösungsmittels - gegebenenfalls unter vermindertem Druck - entfernt. Die verbleibende Lösung wird anschließend abgekühlt - bei Verwendung von Toluol als Lösungsmittel wird vorzugsweise auf eine Temperatur von ca. 20°C abgekühlt. Das auskristallisierte D,L-Lactid wird vom Lösungsmittel - vorzugsweise durch Schleudern - befreit und - gegebenenfalls unter vermindertem Druck - getrocknet.

Das auf diese Art und Weise hergestellte D,L-Lactid kann ohne weitere Reinigungsschritte polymerisiert werden.

Das nachfolgende Beispiel dient zur Erläuterung der Erfindung ohne sie einzuschränken.

### Beispiel

### Herstellung des D,L-Lactids

900 l Toluol werden durch Abdestillieren von ca. 100 l - unter Normaldruck - azeotrop entwässert. Jeweils 135 kg L(-)-Lactid und 135 kg D(+)-Lactid werden in je 400 l azeotrop entwässertem Toluol bei einer Temperatur im Bereich von 40-70°C gelöst. Die beiden Lösungen werden heiß filtriert und anschließend miteinander vermischt. Von der resultierenden Mischung werden bei einem Druck von 50 mbar 160 l Toluol abdestilliert. Die verbleibende Lösung wird auf 20°C abgekühlt, wobei das D,L-Lactid auskristallisiert. Das D,L-Lactid wird abgeschleudert und im Vakuum bei einem Druck von 10-15 mbar getrocknet.

### Man erhält 244,0 kg (90.4 % d. Th.) D,L-Lactid

### Polymerisation von D,L-Lactid

Eine Probe des so erhaltenen D,L-Lactids wird mit einem aus dem Stand der Technik bekannten Katalysator (z.B. Zinn(II)octanoat) polymerisiert. Die Werte der inhärenten Viskosität derartig hergestellter D,L-Lactid-Polymerisate liegen im Bereich von hᵢₙₕ = 4 bis 5 dl/g (gemessen in Chloroform bei 25°C).

## Patentansprüche

1. Verbessertes Verfahren zur Herstellung von D,L-Lactid durch Co-Kristallisation von D-Lactid und L-Lactid, dadurch gekennzeichnet, daß man D-und L-Lactid in einem zur Azeotropbildung mit Wasser befähigten Lösungsmittel oder Lösungsmittelgemisch löst, die Lösung filtriert und - gegebenenfalls unter vermindertem Druck - einengt, die verbleibende Lösung abkühlt, das auskristallisierte D,L-Lactid vom Lösungsmittel befreit und - gegebenenfalls unter vermindertem Druck - trocknet.

2. Verbessertes Verfahren zur Herstellung von D,L-Lactid durch Co-Kristallisation von D-Lactid und L-Lactid nach Anspruch 1, dadurch gekennzeichnet, daß man D- und L-Lactid jeweils in einem zur Azeotropbildung mit Wasser befähigten Lösungsmittel oder Lösungsmittelgemisch löst, beide Lösungen filtriert und vereinigt, die Mischung - gegebenenfalls unter vermindertem Druck - einengt, die verbleibende Lösung abkühlt, das auskristallisierte D,L-Lactid vom Lösungsmittel befreit und - gegebenenfalls unter vermindertem Druck - trocknet.

3. Verbessertes Verfahren zur Herstellung von D,L-Lactid durch Co-Kristallisation von D-Lactid und L-Lactid nach Anspruch 2, dadurch gekennzeichnet, daß man D-Lactid und L-Lactid jeweils in Toluol löst, die Lösungen heiß filtriert und vereinigt, azeotrop entwässert die verbleibende Lösung abkühlt, das auskristallisierte D,L-Lactid vom Lösungsmittel befreit und das Kristallisat unter vermindertem Druck trocknet.

## Claims

1. Improved process for preparing D,L-lactide by cocrystallisation of D-lactide and L-lactide, characterised in that D- and L-lactide are dissolved in a solvent or mixture of solvents capable of forming an azeotrope with water, the solution is filtered and concentrated by evaporation (optionally under reduced pressure), the residual solution is cooled, the D,L-lactide which crystallises out is freed from solvent and dried, optionally under reduced pressure.

2. Improved process for preparing D,L-lactide by cocrystallisation of D-lactide and L-lactide according to claim 1, characterised in that D-lactide and L-lactide are each dissolved in a solvent or mixture of solvents capable of forming an azeotrope with water, the two solutions are filtered and combined, the mixture is concentrated by evaporation (optionally under reduced pressure), the residual solution is cooled, the D,L-lactide which crystallises out is freed from solvent and dried, optionally under reduced pressure.

3. Improved process for preparing D,L-lactide by cocrystallisation of D-lactide and L-lactide according to claim 2, characterised in that D-lactide and L-lactide are each dissolved in toluene, the solutions are filtered while hot and combined, then azeotropically dried, the remaining solution is cooled, the D,L-lactide which crystallises out is freed from solvent and the crystals are dried under reduced pressure.

## Revendications

1. Procédé perfectionné de préparation de D,L-lactide par cocristallisation de D-lactide et de L-lactide, caractérisé en ce que l'on dissout du D-lactide et du L-lactide dans un solvant ou mélange de solvants capable de former un azéotrope avec l'eau, on filtre la solution et on la concentre, éventuellement sous pression réduite, on refroidit la solution restante, on débarrasse le D,L-lactide cristallisé du solvant et on le sèche, éventuellement sous pression réduite.

2. Procédé perfectionné de préparation de D,L-lactide par cocristallisation de D-lactide et de L-lactide selon la revendication 1, caractérisé en ce que l'on dissout du D-lactide et du L-lactide dans un solvant ou mélange de solvants capable de former un azéotrope avec l'eau, on filtre les deux solutions et on les réunit, on concentre le mélange, éventuellement sous pression réduite, on refroidit la solution restante, on débarrasse le D,L-lactide cristallisé du solvant et on le sèche, éventuellement sous pression réduite.

3. Procédé perfectionné de préparation de D,L-lactide par cocristallisation de D-lactide et de L-lactide selon la revendication 2, caractérisé en ce que l'on dissout du D-lactide et du L-lactide dans du toluène, on filtre les solutions à chaud et on les réunit, on élimine l'eau par voie azéotropique, on refroidit la solution restante, on débarrasse le D,L-lactide cristallisé du solvant et on sèche le cristallisat sous pression réduite.
